(19) Europäisches Patentamt
European Patent Office
Office européen des brevets

(11) Veröffentlichungsnummer: **0 496 239 B1**

(12) # EUROPÄISCHE PATENTSCHRIFT

(45) Veröffentlichungstag der Patentschrift: **11.10.95**

(51) Int. Cl.[6]: **C07C 271/22**, A01N 47/12, C07C 271/34, C07C 271/54

(21) Anmeldenummer: **92100416.4**

(22) Anmeldetag: **13.01.92**

(54) **Substituierte Aminosäureamid-Derivate deren Herstellung und Verwendung als Fungizide.**

(30) Priorität: **24.01.91 DE 4102042**

(43) Veröffentlichungstag der Anmeldung:
**29.07.92 Patentblatt 92/31**

(45) Bekanntmachung des Hinweises auf die Patenterteilung:
**11.10.95 Patentblatt 95/41**

(84) Benannte Vertragsstaaten:
**BE CH DE ES FR GB GR IT LI NL**

(56) Entgegenhaltungen:
**EP-A- 0 333 938**
**DE-A- 3 915 755**

**JOURNAL OF MEDICINAL CHEMISTRY, Bd. 11, Nr. 1, 26. Dezember 1967, Washington, US; Seiten 74 - 79, C.HANSCH et al: "The Parabolic Dependence of Drug Action upon Lipophilic Character as Revealed by a Study of Hypnotics"**

**BULLETIN OF THE CHEMICAL SOCIETY OF JAPAN, Bd. 40, Nr. 9, September 1967, Tokyo, JP; Seiten 2164 - 2167, S. SAKAKIBARA et al: "Use of Anhydrous Hydrogen Fluoride in Peptide Synthesis. I. Behavior of Various Protective Groups in Anhydrous Hydrogen Fluoride"**

(73) Patentinhaber: **BAYER AG**

**D-51368 Leverkusen (DE)**

(72) Erfinder: **Seitz, Thomas, Dr.**
**Mozartstrasse 32**
**W-4019 Monheim (DE)**
Erfinder: **Dehne, Heinz-Wilhelm, Dr. habil.**
**Krischer Strasse 81**
**W-4019 Monheim (DE)**

**EP 0 496 239 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Aminosäureamid-Derivate, ein Verfahren zu deren Herstellung sowie deren Verwendung in Schädlingsbekämpfungsmitteln.

Die erfindungsgemäßen Substanzen besitzen eine ausgezeichnete Wirkung bei der Bekämpfung von Schädlingen. Insbesondere können die erfindungsgemäßen Substanzen als Fungizide, vor allem im Pflanzenschutz, verwendet werden.

Bestimmte Aminosäureamide sind bereits bekannt, wie beispielsweise N-tert-Butoxycarbonyl-L-leucylbenzylamid (EP-A-236 874).

Eine Verwendung dieser Verbindungen in Schädlingsbekämpfungsmitteln wird jedoch nicht beschrieben.

Bekannt sind weiterhin bestimmte substituierte Aminosäureamid-Derivate, wie beispielsweise N-(t-Butyloxycarbonyl)-L-valin-phenylethylamid (DE-OS 39 15 755). Die Wirkung dieser Verbindungen ist jedoch, insbesondere bei niedrigen Aufwandmengen und -konzentrationen, nicht befriedigend.

Außderdem ist bekannt, daß bestimmte Diphenylmethylaminosäureamid-Derivate, wie beispielsweise Carbaminsäure-[1-[[(diphenylmethyl)-amino]carbonyl]-2-methylpropyl]-1,1-dimethylethylester pharmakologische Wirksamkeit besitzen (vgl. EP-OS 343 460, EP-OS 247 557).

Gegenstand der Erfindung sind neue substituierte Aminosäureamid-Derivate der allgemeinen Formel (I)

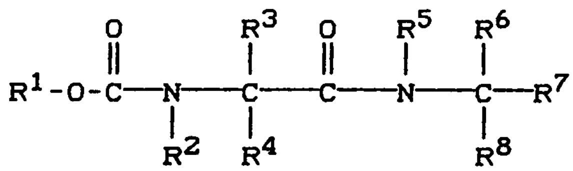

(I).

In dieser Formel steht:

$R^1$

1. für Alkyl, Alkenyl, Alkinyl, Halogenalkyl, Halogenalkenyl, Halogenalkinyl, Cycloalkyl, Cycloalkenyl, unsubstituiertes oder substituiertes Aryl und unsubstituiertes oder substituiertes Heteroaryl,

2. vorzugsweise für geradkettiges oder verzweigtes Alkyl mit 1 bis 6 Kohlenstoffatomen, geradkettiges oder verzweigtes Alkenyl oder Alkinyl mit 2 bis 6 Kohlenstoffatomen, geradkettiges oder verzweigtes Halogenalkyl mit 1 bis 6 Kohlenstoffatomen und 1 bis 9 gleichen oder verschiedenen Halogenatomen, geradkettiges oder verzweigtes Halogenalkenyl oder Halogenalkinyl mit 2 bis 6 Kohlenstoffatomen und 1 bis 9 gleichen oder verschiedenen Halogenatomen, für substituiertes oder unsubstituiertes Cycloalkyl oder Cycloalkenyl mit 3 bis 7 Kohlenstoffatomen, für jeweils unsubstituiertes oder substituiertes Phenyl oder Pyridyl, wobei als Substituenten im Pyridyl-bzw. Phenylteil jeweils in Frage kommen: Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 Halogenatomen, wobei die Halogenatome gleich oder verschieden sind; Halogen; Alkylalkoxy mit 1 bis 4 Kohlenstoffatomen in jedem Alkylteil; Carbonylalkoxy mit 1 bis 4 Kohlenstoffatomen im Alkylteil;

3. besonders bevorzugt für geradkettiges oder verzweigtes Alkyl mit 1 bis 4 Kohlenstoffatomen, geradkettiges oder verzweigtes Alkenyl oder Alkinyl mit 2 bis 4 Kohlenstoffatomen, geradkettiges oder verzweigtes Halogenalkyl mit 1 bis 4 Kohlenstoffatomen und 1 bis 5 gleichen oder verschiedenen Halogenatomen, geradkettiges oder verzweigtes Halogenalkenyl oder Halogenalkinyl mit 2 bis 4 Kohlenstoffatomen und 1 bis 5 gleichen oder verschiedenen Halogenatomen, für unsubstituiertes oder substituiertes Cycloalkyl oder Cycloalkenyl mit 3 bis 6 Kohlenstoffatomen, für unsubstituiertes oder einfach bis dreifach, gleich oder verschieden substituiertes Phenyl steht, wobei die folgenden Substituenten in Frage kommen: Alkyl, Alkoxy und Alkylthio mit jeweils 1 bis 4 Kohlenstoffatomen; Halogenalkyl, Halogenalkoxy und Halogenalkylthio mit 1 oder 2 Kohlenstoffatomen und 1 bis 5 Halogenatomen, wobei die Halogenatome gleich oder verschieden sind; Fluor, Chlor, Brom und Iod; Alkylalkoxy mit 1 bis 2 Kohlenstoffatomen in jedem Alkylteil; Carbonylalkoxy mit 1 oder 2 Kohlenstoffatomen im Alkylteil;

4. ganz besonders bevorzugt für Methyl, Ethyl, n- und i-Propyl, n-, i-, s- und t-Butyl, Fluormethyl, Fluorethyl, Fluorpropyl, Chlorpropyl, Fluorbutyl, Chlorbutyl, Difluormethyl, Difluorpropyl, Dichlorpropyl, Difluorbutyl, Dichlorbutyl, Trifluormethyl, Trichlormethyl, Trifluorethyl, Trichlorethyl, Trifluorpropyl, Trichlorpropyl, Trifluorbutyl, Trichlorbutyl, Allyl, Butenyl, Propargyl, Butinyl, Fluor- oder Chlorallyl, -butenyl, -propargyl, -butinyl, Cyclopropyl, Cyclopentyl, Cyclohexyl, Cyclopentenyl oder Cyclohexenyl, für unsubstituiertes oder einfach bis zweifach, gleich oder verschieden substituiertes Phenyl, wobei die folgenden Substituenten in Frage kommen: Methyl, Ethyl, n- und i-Propyl, n-, i-, s-und t-Butyl, Methoxy, Ethoxy, i- und n-Propoxy, n-, i-, s- und t-Butoxy, Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Chlor, Brom, Fluor.

$R^2$ und $R^5$ stehen unabhängig voneinander
1. für Wasserstoff oder Alkyl;
2. vorzugsweise für Wasserstoff oder Methyl;
3. besonders bevorzugt für Wasserstoff.

$R^3$ und $R^4$ stehen unabhängig voneinander
1. für Wasserstoff, Cycloalkyl oder Alkyl oder bilden zusammen mit dem Kohlenstoffatom, an das sie gebunden sind, einen Cycloalkylring;
2. vorzugsweise für Wasserstoff, unsubstituiertes oder substituiertes Cycloalkyl mit 3 bis 7 Kohlenstoffatomen oder geradkettiges oder verzweigtes Alkyl mit 1 bis 6 Kohlenstoffatomen oder bilden zusammen mit dem Kohlenstoffatom, an das sie gebunden sind, einen Cycloalkylring mit 3 bis 7 Kohlenstoffatomen;
3. besonders bevorzugt für Wasserstoff unsubstituiertes oder substituiertes Cycloalkyl mit 3 bis 6 Kohlenstoffatomen oder geradkettiges oder verzweigtes Alkyl mit 1 bis 5 Kohlenstoffatomen oder bilden zusammen mit dem Kohlenstoffatom, an das sie gebunden sind, einen Cycloalkylring mit 3 bis 6 Kohlenstoffatomen;
4. ganz besonders bevorzugt für Wasserstoff, Methyl, Ethyl, n- oder i-Propyl, n-, i-, s- oder t-Butyl, 3-Pentyl, Cyclopropyl, Cyclopentyl oder Cyclohexyl oder bilden zusammen mit dem Kohlenstoffatom, an das sie gebunden sind, einen Cyclopropyl-, Cyclopentyl- oder Cyclohexylring.

$R^6$ steht
1. für Wasserstoff oder Alkyl,
2. vorzugsweise für Wasserstoff oder verzweigtes oder unverzweigtes Alkyl mit 1 bis 6 Kohlenstoffatomen,
3. besonders bevorzugt für Wasserstoff oder verzweigtes oder unverzweigtes Alkyl mit 1 bis 4 Kohlenstoffatomen,
4. ganz besonders bevorzugt für Wasserstoff, Methyl, Ethyl, n- und iso-Propyl.

$R^7$ und $R^8$ stehen unabhängig voneinander
1. für jeweils unsubstituiertes oder substituiertes Phenyl oder Heteroaryl,
2. vorzugsweise für jeweils unsubstituiertes oder substituiertes Phenyl, Pyridyl, Furanyl oder Thienyl, wobei als Substituenten jeweils in Frage kommen: Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 Halogenatomen, wobei die Halogenatome gleich oder verschieden sind; Hydroxy; Halogen; Cyano; Nitro; Dialkylamino mit 1 bis 4 Kohlenstoffatomen je Alkylgruppe; Carboxy; Alkylalkoxy mit 1 bis 4 Kohlenstoffatomen in jedem Alkylteil; Carbonylalkoxy mit 1 bis 4 Kohlenstoffatomen im Alkylteil; Carbonylalkyl mit 1 bis 4 Kohlenstoffatomen in Alkylteil; Formyl; Carbonylaryloxy mit 6 bis 10 Kohlenstoffatomen im Arylteil; Carbonylaryl mit 6 bis 10 Kohlenstoffatomen im Arylteil; Oxycarbonylalkyl mit 1 bis 4 Kohlenstoffatomen im Alkylteil; Oxycarbonylaryl mit 6 bis 10 Kohlenstoffatomen im Arylteil; Carbonylamino, Carbonylaminoalkyl, Carbonylaminodialkyl, Aminocarbonyl, Alkylaminocarbonyl, Aminocarbonylalkyl und Alkylaminocarbonylalkyl mit jeweils 1 bis 4 Kohlenstoffatomen im Alkylteil; Sulfonamido; Sulfonylalkyl und Sulfonylalkoxy mit jeweils 1 bis 4 Kohlenstoffatomen; jeweils unsubstituiertes oder durch Halogen substituiertes Phenyl oder Phenoxy,
3. besonders bevorzugt, für jeweils unsubstituiertes oder substituiertes Phenyl, Pyridyl, Thienyl oder Furanyl, wobei als Substituenten in Frage kommen: Alkyl, Alkoxy und

Alkylthio mit jeweils 1 oder 2 Kohlenstoffatomen; Halogenalkyl, Halogenalkoxy und Halogenalkylthio mit 1 oder 2 Kohlenstoffatomen und 1 bis 5 Halogenatomen, wobei die Halogenatome gleich oder verschieden sind; Hydroxy; Fluor, Chlor, Brom und Jod; Cyano; Nitro; Dialkylamino mit 1 oder 2 Kohlenstoffatomen je Alkylgruppe; Carboxy; Alkylalkoxy mit 1 oder 2 Kohlenstoffatomen in jedem Alkylteil; Carbonylalkoxy mit 1 oder 2 Kohlenstoffatomen im Alkylteil; Carbonylalkyl mit 1 oder 2 Kohlenstoffatomen im Alkylteil; Formyl; Carbonylphenoxy; Benzoyl; Oxycarbonylalkyl mit 1 oder 2 Kohlenstoffatomen; Benzoyloxy; Carbonylamino, Carbonylaminoalkyl, Carbonylaminodialkyl, Aminocarbonyl, Alkylaminocarbonyl, Aminocarbonylalkyl und Alkylaminocarbonylalkyl mit jeweils 1 oder 2 Kohlenstoffatomen im Alkylteil; Sulfonamido; Sulfonylalkyl und Sulfonylalkoxy mit jeweils 1 oder 2 Kohlenstoffatomen; jeweils unsubstituiertes oder durch Fluor, Chlor oder Brom substituiertes Phenyl oder Phenoxy,

4. ganz besonders bevorzugt für jeweils unsubstituiertes oder substituiertes Phenyl, Pyridyl, Furanyl oder Thienyl, wobei als Phenylsubstituenten Methyl, Ethyl, n- und i-Propyl, n-, i-, s- und t-Butyl, Methoxy, Ethoxy, i- und n-Propoxy, n-, i-, s- und t-Butoxy, Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Chlor, Brom, Fluor, Nitro und Cyano und als Heteroarylsubstituenten Chlor, Fluor, Brom und Methyl in Frage kommen.

Ausgenommen ist die Verbindung Carbaminsäure-[1-[[(diphenylmethyl)-amino]-carbonyl]-2-methylpropyl]-1,1-dimethylethylester.

Die hier aufgeführten Definitionen der einzelnen Reste gelten in entsprechender Weise auch für alle Kombinationen der Reste in der Verbindung der Formel (I) sowie für die Zwischen- und Vorprodukte.

Hierbei werden die Reste $R^1$ bis $R^8$ im Rahmen ihrer jeweils angegebenen, allgemeinen und bevorzugten Definitionen beliebig kombiniert (siehe Vorzugsbereiche 1. bis 4.)

Von besonderem Interesse sind diejenigen Kombinationen der Reste $R^1$ bis $R^8$ aus den jeweils gleichen Vorzugsbereichen, insbesondere aber die Kombination der Reste $R^1$, $R^3$, $R^4$, $R^6$, $R^7$ und $R^8$ im Vorzugsbereich 4. mit den Resten $R^2$ und $R^5$ im Vorzugsbereich 3..

Vor allem bevorzugt sind die substituierten Aminosäureamid-Derivate der allgemeinen Formel (I), in welcher

$R^1$, $R^2$, $R^5$, $R^6$, $R^7$ und $R^8$ die oben, insbesondere die in den Vorzugsbereichen, angegebenen Bedeutungen haben und

$R^3$ für Methyl, Ethyl, n-Propyl, t-Butyl, i-Butyl, 3-Pentyl, Cyclopentyl oder Cyclohexyl insbesondere für i-Propyl, s-Butyl oder Cyclopentyl steht,

$R^4$ für Wasserstoff steht oder

$R^3$ und $R^4$ zusammen mit dem Kohlenstoffatom, an das sie gebunden sind, einen Cycloalkylring mit 3 bis 6 Kohlenstoffatomen, insbesondere einen Cyclopropyl-, Cyclopentyl- oder Cyclohexylring, bilden,

ausgenommen die Verbindung Carbaminsäure-[1-[[(diphenylmethyl)-amino]-carbonyl]-2-methylpropyl]-1,1-dimethylethylester.

Die erfindungsgemäß zu verwendenden substituierten Aminosäureamid-Derivate der Formel (I) ist teilweise bekannt (vgl. EP-OS 343 460 und EP-OS 247 557). Eine Verwendung dieser Verbindung in Schadlingsbekämpfungsmitteln wird jedoch nicht beschrieben.

Gegenstand der vorliegenden Anmeldung ist daher auch die Verwendung der oben beschriebenen neuen Verbindungen der Formel (I) und der bekannten Verbindung Carbaminsäure-[1-[[(diphenylmethyl)-amino]-carbonyl]-2-methylpropyl]-1,1-dimethylethylester als Fungizide.

Überraschenderweise zeigen die erfindungsgemäß zu verwendenden substituierten Aminosäureamid-Derivate der Formel (I) eine bessere fungizide Wirksamkeit, als das aus dem Stand der Technik bekannte N-(t-Butyloxycarbonyl)-L-valin-phenyl-ethylamid.

Man erhält die substituierten Aminosäureamid-Derivate der allgemeinen Formel (I)

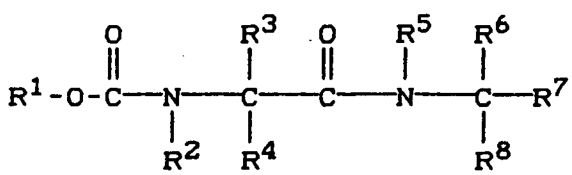

(I),

in welcher

$R^1$, $R^2$, $R^3$, $R^4$, $R^5$, $R^6$, $R^7$ und $R^8$ die oben angegebene Bedeutung haben,

wenn man

substituierte Aminosäure der Formel (II)

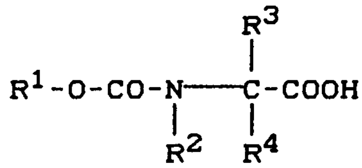

(II),

in welcher

$R^1$, $R^2$, $R^3$ und $R^4$ die oben angegebene Bedeutung haben, bzw. deren carboxy-aktivierte Derivate, gegebenenfalls in Gegenwart eines Katalysators, gegebenenfalls in Gegenwart eines Säurebindemittels und gegebenenfalls in Gegenwart eines Verdünnungsmittels

mit einem Amin der Formel (III)

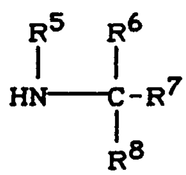

(III)

in welcher

$R^5$, $R^6$, $R^7$ und $R^8$ die oben angegebene Bedeutung haben,

umsetzt.

Die bekannte Verbindung kann in Analogie zu dem Verfahren zur Herstellung der Verbindungen der Formel (I) erhalten werden.

Die bekannte Verbindung und die Verbindungen der Formel (I) können außerdem ein oder mehrere Chiralitätszentren enthalten und können somit in verschiedenen Enantiomeren- und Diastereomerengemischen vorliegen, die gegebenenfalls in üblicher Art und Weise getrennt werden können. Sowohl die reinen Enantiomeren und Diastereomeren, als auch die Gemische werden erfindungsgemäß beansprucht.

Im folgenden wird der Einfachheit halber stets von Verbindungen der Formel (I) gesprochen, obwohl sowohl die reinen Verbindungen, als auch die Gemische mit unterschiedlichen Anteilen an isomeren, enantiomeren und diastereomeren Verbindungen gemeint sind.

Verwendet man beispielsweise i-Propoxycarbonyl-L-valin und $\alpha$-Aminodiphenylmethan als Ausgangsstoffe, so kann der Ablauf des erfindungsgemäßen Verfahrens durch das folgende Formelschema wiedergegeben werden:

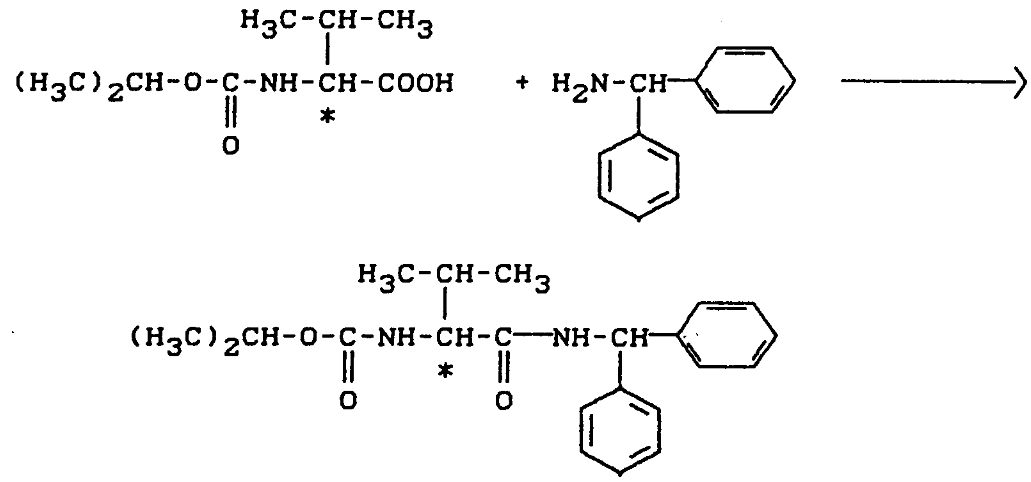

Die Aminosäure-Derivate der Formel (II) sind allgemein bekannt (vgl. z.B. Houben-Weyl, Methoden der organischen Chemie, Band XV, Teil 1 und 2, Seiten 46 ff und 112 ff, Georg Thieme Verlag, Stuttgart 1974; D. Keller et.al., Org. Synth. 60, 2145 (1981); bzw. R.C. Sheppard, A Specialist Periodical Report, Aminoacids, Peptids and Proteins, The Royal Society of Chemistry, Burlington House, London 1978, bzw. I.P. Greenstein and M Winitz, Chemistry of Amino Acids, I. Wiley Sons Inc., New York, London 1961; bzw. E. Schröder and K. Lübke, The Peptides Vol. I, Academic Press, New York, London 1965) oder können nach den dort angegebenen Verfahren erhalten werden.

Die außerdem für die Durchführung des erfindungsgemäßen Verfahrens als Ausgangsstoffe zu verwendenden carboxyaktivierten Derivate der Aminosäure der Formel (II) sind allgemein bekannt.

Als carboxy-aktivierte Derivate der Aminosäuren der Formel (II) kommen alle Carboxy-aktivierten Derivate infrage, wie Säurehalogenide, wie z.B. Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxisuccinimidester sowie mit Kondensationsmitteln, wie z.B. Dicyclohexylcarbodiimid oder Carbonyldiimidazol, in situ erzeugte aktivierte Formen der Aminosäuren.

Vorzugsweise werden die den Aminosäuren der Formel (II) entsprechenden Säurechloride und gemischten Anhydride eingesetzt. Sie können hergestellt werden, indem man die Aminosäuren der Formel (II) oder deren Salze mit einem Halogenierungsmittel oder einem der allgemein bekannten Mittel zur Herstellung von gemischten Anhydriden, wie beispielsweise Phosphorpentachlorid, Thionylchlorid, Oxalylchlorid oder Chlorameisensäureisobutylester, in allgemein bekannter Art und Weise umsetzt. Bevorzugt ist der Einsatz von Chlorameisensäureisobutylester.

Diese Umsetzung kann in Gegenwart indifferenter Verdünnungsmittel wie z.B. aromatische, nichtaromatische oder halogenierte Kohlenwasserstoffe wie: Ketone, wie z.B. Aceton; Ester, wie z.B. Ethylacetat; Amide, wie z.B. Dimethylformamid-; Nitrile, wie z.B. Acetonitril; Chlorkohlenwasserstoffe, wie z.B. Methylenchlorid; Kohlenwasserstoffe, wie z.B. Toluol; oder Ether, wie z.B. Tetrahydrofuran bzw. deren Mischungen und/oder in Gegenwart eines Säurebindemittels, wie vorzugsweise eines tertiären Amins, wie z.B. Triethylamin, Pyridin oder N-Methylpiperidin, bei Temperaturen von -78 °C bis 100 °C, vorzugsweise -60 °C bis 25 °C, durchgeführt werden.

Die Amine der Formel (III) sind allgemein bekannte Verbindungen der organischen Chemie.

Als Verdünnungsmittel kommen für das erfindungsgemäße Verfahren inerte, organische Lösungsmittel wie: Ketone, wie Aceton oder Ethylmethylketon; Ester wie Ethyl- oder Methylacetat; Amide wie Dimethylformamid; Nitrile, wie Acetonitril; Chlorkohlenwasserstoffe, wie Methylenchlorid oder Tetrachlorkohlenstoff; Kohlenwasserstoffe, wie Toluol oder Ether, wie Tetrahydrofuran sowie gegebenenfalls Wasser und deren Mischungen in Frage.

Als Säurebindemittel kommen für das erfindungsgemäße Verfahren übliche anorganische und organische Säurebinder in Frage. Hierzu gehören vorzugsweise tertiäre Amine, wie Triethylamin, Pyridin oder N-Methylpiperidin, sowie anorganische Basen, z.B. Metallhydroxide wie Natrium- und Kaliumhydroxid Metallcarbonate wie Natriumcarbonat oder Calciumcarbonat.

Das erfindungsgemäße Verfahren wird gegebenenfalls in Gegenwart eines Katalysators durchgeführt. Beispielsweise genannt seien 4-Dimethylaminopyridin, 1-Hydroxy-benzotriazol oder Dimethylformamid.

Die Temperaturen können bei der Durchführung des Verfahrens in einem größeren Bereich variiert werden. Im allgemeinen arbeitet man bei Temperaturen von -78 bis +120 °C, vorzugsweise bei -60 bis +40 °C.

Bei der Durchführung des erfindungsgemäßen Verfahrens arbeitet man vorzugsweise in äquimolaren Mengen.

Dabei werden die Aminosäurederivate der Formel (II) als reine optische Isomere (D bzw. L-Form) oder als Racemate eingesetzt.

Die Erfindung umfaßt sowohl die reinen Isomeren als auch die Gemische. Diese Gemische können nach gebräuchlichen Methoden, z.B. selektive Kristallisation aus geeigneten Lösungsmitteln oder Chromatographie an Kieselgel oder Aluminiumoxid in die Komponenten aufgetrennt werden. Racemate können nach üblichen Methoden in die einzelnen Enantiomeren aufgetrennt werden, so z.B. durch Salzbildung mit optisch aktiven Säuren wie Champhersulfonsäure oder Dibenzoylweinsäure und selektive Kristallisation oder durch Derivatisierung mit geeigneten, optisch aktiven Reagenzien, Trennung der diastereomeren Derivate und Rückspaltung oder Trennung an optisch aktivem Säulenmaterial.

Die erfindungsgemäßen und bekannten Wirkstoffe der Formel (I) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg protektiv zur Bekämpfung von Phytophthora-Arten an Tomaten oder Plasmopara-Arten an Reben einsetzen.

Die Wirkstoffe werden in Abhängigkeit von ihren jeweiligen physikalischen und/oder chemischen Eigenschaften vorzugsweise in übliche Formulierungen überführt, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut von Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die Herstellung und die Verwendung der erfindungsgemäßen Wirkstoffe wird durch die folgenden Beispiele illustriert.

Herstellungsbeispiele

Beispiel 1

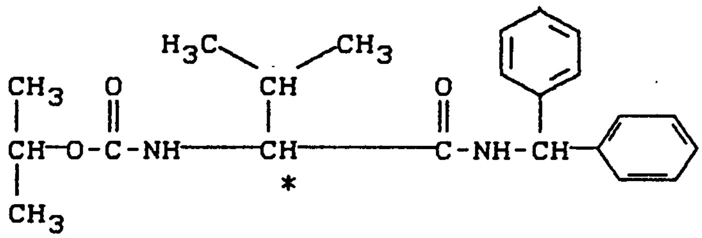

Zu 4,67 g i-Propoxycarbonyl-L-valin (0,023 Mol), gelöst in 50 ml $CH_2Cl_2$, wurden bei -20 °C 2,3 g (0,023 Mol) N-Methylpiperidin zugegeben. Anschließend tropft man bei -20 °C schnell 3,2 g (0,023 Mol) Chlorameisensäureisobutylester zu, rührt bei gleicher Temperatur 10 Minuten nach, kühlt auf -60 °C ab und läßt 4,21 g (0,023 Mol) α-Aminodiphenylmethan gelöst in 10 ml Dichlormethan zulaufen, wobei die Temperatur unter -15 °C gehalten wird, Nach 2 Stunden bei -15 °C läßt man 15 Stunden bei Raumtemperatur nachrühren, filtriert vom Feststoff ab, wäscht mit $CH_2Cl_2$ nach, engt ein, gibt den Rückstand auf Wasser, extrahiert 2mal mit Essigester, wäscht die vereinigten Essigester-Phasen mit $NaHCO_3$-Lösung und Wasser, trocknet und engt ein, Man erhält 5,5 g (66 % d, Th.) N-(i-Propyloxycarbonyl)-L-valin-diphenylmethylamid mit einem Schmelzpunkt von 145 °C.

Analog Beispiel 1 werden die folgenden Verbindungen der Formel (I) erhalten

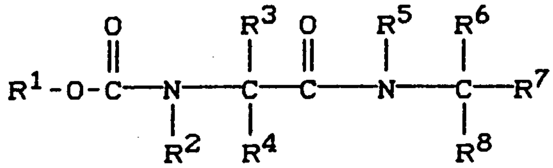

(I),

Tabelle 1

| Bsp. Nr. | $R^1$ | $R^2$ | $R^3$ | $R^4$ | $R^5$ | $R^6$ | $R^7$ | $R^8$ | physikal. Konstanten |
|---|---|---|---|---|---|---|---|---|---|
| 2 | $-CH(CH_3)_2$ | H | $-CH(CH_3)_2$ | H | H | H | 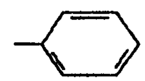 | 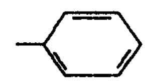 | Fp: 164° C |
| 3 | $s-C_4H_9$ | H | $-CH(CH_3)_2$ | H | H | H | 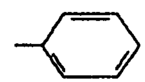 | 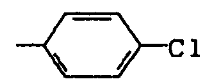 | Fp: 154° C |
| 4 | $-CH(CH_3)_2$ | H | $-CH(CH_3)_2$ | H | H | H | 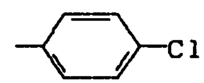 | 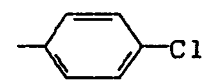 | Fp: 184° C |
| 5 | $s-C_4H_9$ | H | $-CH(CH_3)_2$ | H | H | H | 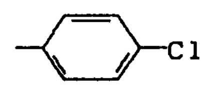 | 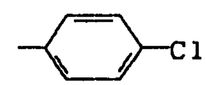 | Fp: 167° C |
| 6 | $-CH(CH_3)_2$ | H | $-CH(CH_3)_2$ | H | H | H | 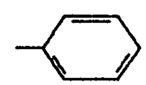 | 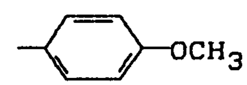 | Fp: 162° C |

**Tabelle 1** (Fortsetzung)

| Bsp. Nr. | $R^1$ | $R^2$ | $R^3$ | $R^4$ | $R^5$ | $R^6$ | $R^7$ | $R^8$ | physikal. Konstanten |
|---|---|---|---|---|---|---|---|---|---|
| 7 | $s-C_4H_9$ | H | $-CH(CH_3)_2$ | H | H | H | 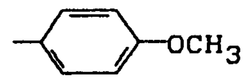 | 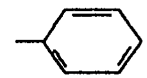 | Fp: 115° C |
| 8 | $-CH(CH_3)_2$ | H | $-CH(CH_3)_2$ | H | H | H | 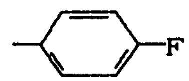 | 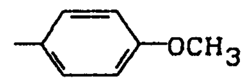 | Fp: 188° C |
| 9 | $s-C_4H_9$ | H | $-CH(CH_3)_2$ | H | H | H | 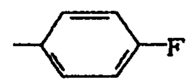 | 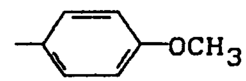 | Fp: 152° C |
| 10 | 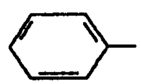 | H | $-CH(CH_3)_2$ | H | H | H | 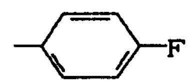 | 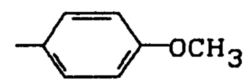 | Fp: 124° C |
| 11 | 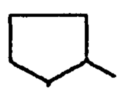 | H | $-CH(CH_3)_2$ | H | H | H | 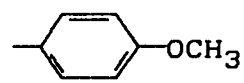 | 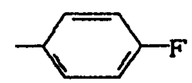 | Fp: 156° C |

**Tabelle 1** (Fortsetzung)

| Bsp. Nr. | $R^1$ | $R^2$ | $R^3$ | $R^4$ | $R^5$ | $R^6$ | $R^7$ | $R^8$ | physikal. Konstanten |
|---|---|---|---|---|---|---|---|---|---|
| 12 | $-CH(CH_3)_2$ | H | $-CH(CH_3)_2$ | H | H | H | 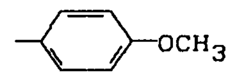 | 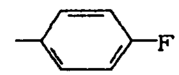 | Fp: 160° C |
| 13 | $-CH(CH_3)_2$ | H | $-CH(CH_3)_2$ | H | H | H | 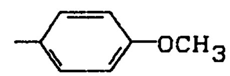 | 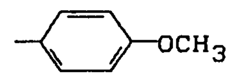 | Fp: 204° C |
| 14 | $s-C_4H_9$ | H | $-CH(CH_3)_2$ | H | H | H | 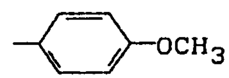 | 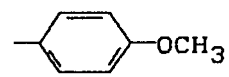 | Fp: 195° C |
| 15 | 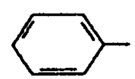 | H | $-CH(CH_3)_2$ | H | H | H | 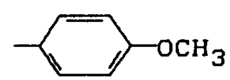 | 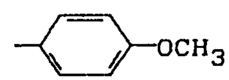 | Fp: 200° C |
| 16 | 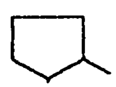 | H | $-CH(CH_3)_2$ | H | H | H | 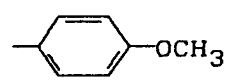 | 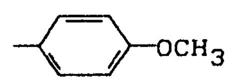 | Fp: 197° C |
| 17 | $-CH(CH_3)_2$ | H | $-CH_3-CH-C_2H_5$ | H | H | H | 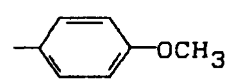 | 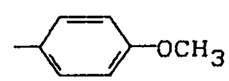 | Fp: 218° C |

Anwendungsbeispiele:

In den folgenden Anwendungsbeispielen wurde die nachfolgend aufgeführte Verbindung als Vergleichssubstanz eingesetzt:

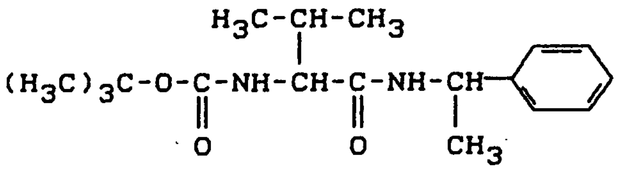

(A)

(bekannt aus DE-OS 39 15 755) N-(t-Butyloxycarbonyl)-L-valin-phenyl-ethylamid

Beispiel A

Phytophthora-Test (Tomate)/protektiv

| | |
|---|---|
| Lösungsmittel: | 4,7 Gewichtsteile Aceton |
| Emulgator: | 0,3 Gewichtsteile Alkyl-arylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20 °C aufgestellt.

3 Tage nach der Inokulation erfolgt die Auswertung.

Eine bis zu 100 Wirkungsgrade höhere Wirksamkeit gegenüber dem Stand der Technik zeigen in diesem Test bei einer Wirkstoffkonzentration von 10 ppm z.B. die Verbindungen gemäß folgender Herstellungsbeispiele: (1), (2) und (3).

Beispiel B

Plasmopara-Test (Reben) / protektiv

| | |
|---|---|
| Lösungsmittel: | 4,7 Gewichtsteile Aveton |
| Emulgator: | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca, 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine bis zu 100 Wirkungsgrade höher Wirksamkeit gegenüber dem Stand der Technik zeigen in diesem Test bei einer Wirkstoffkonzentration von 10 ppm z.B. die Verbindungen gemäß folgender Herstellungsbeispiele: (1), (2) und (3).

## Patentansprüche

1. Verwendung der Aminosäureamid-Derivate der allgemeinen Formel (I)

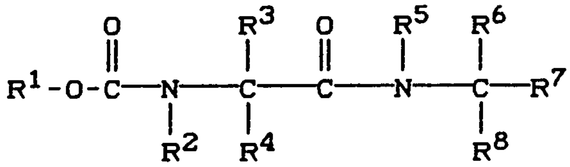

(I),

in welcher

| | |
|---|---|
| $R^1$ | für Alkyl, Alkenyl, Alkinyl, Halogenalkyl, Halogenalkenyl, Halogenalkinyl, Cycloalkyl, Cycloalkenyl, unsubstituiertes oder substituiertes Aryl und unsubstituiertes oder substituiertes Heteroaryl, steht, |
| $R^2$, $R^5$ und $R^6$ | gleich oder verschieden sind und für Wasserstoff oder Alkyl stehen, |
| $R^3$ und $R^4$ | für Wasserstoff, Cycloalkyl oder Alkyl stehen oder |
| $R^3$ und $R^4$ | zusammen mit dem Kohlenstoffatom, an das sie gebunden sind, einen Cycloalkylring bilden und |
| $R^7$ und $R^8$ | gleich oder verschieden sind und jeweils unabhängig voneinander für jeweils unsubstituiertes oder substituiertes Phenyl oder Heteroaryl stehen, |

als Fungizide.

2. Aminosäureamid-Derivate der allgemeinen Formel (I)

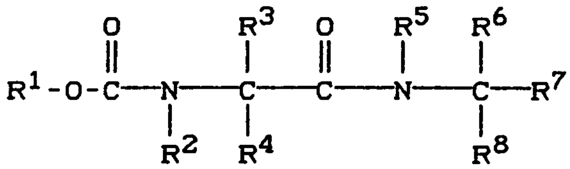

(I),

in welcher

| | |
|---|---|
| $R^1$ | für Alkyl, Alkenyl, Alkinyl, Halogenalkyl, Halogenalkenyl, Halogenalkinyl, Cycloalkyl, Cycloalkenyl, unsubstituiertes oder substituiertes Aryl und unsubstituiertes oder substituiertes Heteroaryl steht, |
| $R^2$, $R^5$ und $R^6$ | gleich oder verschieden sind und für Wasserstoff oder Alkyl stehen, |
| $R^3$ und $R^4$ | für Wasserstoff, Cycloalkyl oder Alkyl stehen oder |
| $R^3$ und $R^4$ | zusammen mit dem Kohlenstoffatom, an das sie gebunden sind, einen Cycloalkylring bilden und |
| $R^7$ und $R^8$ | gleich oder verschieden sind und jeweils unabhängig voneinander für jeweils unsubstituiertes oder substituiertes Phenyl oder Heteroaryl stehen, |

ausgenommen die Verbindung Carbaminsäure-[1-[[(diphenylmethyl)-amino]carbonyl]-2-methylpropyl]-1,1-dimethylethylester.

3. Verbindungen der Formel (I) gemäß Anspruch 2, in welcher

| | |
|---|---|
| $R^1$ | für geradkettiges oder verzweigtes Alkyl mit 1 bis 6 Kohlenstoffatomen, geradkettiges oder verzweigtes Alkenyl oder Alkinyl mit 2 bis 6 Kohlenstoffatomen, geradkettiges oder verzweigtes Halogenalkyl mit 1 bis 6 Kohlenstoffatomen und 1 bis 9 gleichen oder verschiedenen Halogenatomen, geradkettiges oder verzweigtes Halogenalkenyl oder Halogenalkinyl mit 2 bis 6 Kohlenstoffatomen und 1 bis 9 gleichen oder verschiedenen Halogenatomen oder substituiertes oder unsubstituiertes Cycloalkyl oder Cycloalkenyl mit 3 bis 7 Kohlenstoffatomen steht, für jeweils unsubstituiertes oder substituiertes Phenyl und Pyridyl stehen, wobei als Substituenten im Pyridyl- bzw. Phenylteil jeweils in Frage kommen: Alkyl, Alkoxy und Alkylthio mit jeweils 1 bis 4 Kohlenstoffatomen; Halogenalkyl, Halogenalkoxy und Halogenalkylthio mit jeweils 1 |

bis 4 Kohlenstoffatomen und 1 bis 9 Halogenatomen, wobei die Halogenatome gleich oder verschieden sind; Halogen; Alkylalkoxy mit 1 bis 4 Kohlenstoffatomen in jedem Alkylteil; Carbonylalkoxy mit 1 bis 4 Kohlenstoffatomen im Alkylteil;

$R^2$ und $R^5$ für Wasserstoff oder Methyl stehen

$R^3$ und $R^4$ gleich oder verschieden sind und für Wasserstoff, unsubstituiertes oder substituiertes Cycloalkyl mit 3 bis 7 Kohlenstoffatomen oder geradkettiges oder verzweigtes Alkyl mit 1 bis 6 Kohlenstoffatomen stehen oder

$R^3$ und $R^4$ zusammen mit dem Kohlenstoffatom, an das sie gebunden sind, einen Cycloalkylring mit 3 bis 7 Kohlenstoffatomen bilden,

$R^6$ für Wasserstoff oder verzweigtes oder unverzweigtes Alkyl mit 1 bis 6 Kohlenstoffatomen steht und

$R^7$ und $R^8$ gleich oder verschieden sind und für jeweils unsubstituiertes oder substituiertes Phenyl, Pyridyl, Furanyl oder Thienyl stehen, wobei als Substituenten jeweils in Frage kommen:

Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 Halogenatomen, wobei die Halogenatome gleich oder verschieden sind; Hydroxy; Halogen; Cyano; Nitro; Dialkylamino mit 1 bis 4 Kohlenstoffatomen je Alkylgruppe; Carboxy; Alkylalkoxy mit 1 bis 4 Kohlenstoffatomen in jedem Alkylteil; Carbonylalkoxy mit 1 bis 4 Kohlenstoffatomen im Alkylteil; Carbonylalkyl mit 1 bis 4 Kohlenstoffatomen in Alkylteil; Formyl; Carbonylaryloxy mit 6 bis 10 Kohlenstoffatomen im Arylteil; Carbonylaryl mit 6 bis 10 Kohlenstoffatomen im Arylteil; Oxycarbonylalkyl mit 1 bis 4 Kohlenstoffatomen im Alkylteil; Oxycarbonylaryl mit 6 bis 10 Kohlenstoffatomen im Arylteil; Carbonylamino, Carbonylaminoalkyl, Carbonylaminodialkyl, Aminocarbonyl, Alkylaminocarbonyl, Aminocarbonylalkyl und Alkylaminocarbonylalkyl mit jeweils 1 bis 4 Kohlenstoffatomen im Alkylteil; Sulfonamido; Sulfonylalkyl und Sulfonylalkoxy mit jeweils 1 bis 4 Kohlenstoffatomen; jeweils unsubstituiertes oder durch Halogen substituiertes Phenyl oder Phenoxy,

ausgenommen die Verbindung Carbaminsäure-[1-[[(diphenylmethyl)-amino]carbonyl]-2-methylpropyl]-1,1-dimethylethylester.

**4.** Verbindungen der Formel (I) gemäß Anspruch 2, in welcher

$R^1$ für geradkettiges oder verzweigtes Alkyl mit 1 bis 4 Kohlenstoffatomen, geradkettiges oder verzweigtes Alkenyl oder Alkinyl mit 2 bis 4 Kohlenstoffatomen, geradkettiges oder verzweigtes Halogenalkyl mit 1 bis 4 Kohlenstoffatomen und 1 bis 5 gleichen oder verschiedenen Halogenatomen, geradkettiges oder verzweigtes Halogenalkenyl oder Halogenalkinyl mit 2 bis 4 Kohlenstoffatomen und 1 bis 5 gleichen oder verschiedenen Halogenatomen oder unsubstituiertes oder substituiertes Cycloalkyl oder Cycloalkenyl mit 3 bis 6 Kohlenstoffatomen steht und weiterhin für unsubstituiertes oder einfach bis dreifach, gleich oder verschieden substituiertes Phenyl steht, wobei die folgenden Substituenten in Frage kommen: Alkyl, Alkoxy und Alkylthio mit jeweils 1 bis 4 Kohlenstoffatomen; Halogenalkyl, Halogenalkoxy und Halogenalkylthio mit 1 oder 2 Kohlenstoffatomen und 1 bis 5 Halogenatomen, wobei die Halogenatome gleich oder verschieden sind; Fluor, Chlor, Brom und Iod; Alkylalkoxy mit 1 bis 2 Kohlenstoffatomen in jedem Alkylteil; Carbonylalkoxy mit 1 oder 2 Kohlenstoffatomen im Alkylteil;

$R^2$ und $R^5$ für Wasserstoff stehen,

$R^3$ und $R^4$ gleich oder verschieden sind und für Wasserstoff unsubstituiertes oder substituiertes Cycloalkyl mit 3 bis 6 Kohlenstoffatomen oder geradkettiges oder verzweigtes Alkyl mit 1 bis 5 Kohlenstoffatomen stehen oder

$R^3$ und $R^4$ zusammen mit dem Kohlenstoffatom, an das sie gebunden sind, einen Cycloalkylring mit 3 bis 6 Kohlenstoffatomen bilden

$R^6$ für Wasserstoff oder verzweigtes oder unverzweigtes Alkyl mit 1 bis 4 Kohlenstoffatomen steht und

$R^7$ und $R^8$ gleich oder verschieden sind und jeweils unabhängig voneinander für jeweils unsubstituiertes oder substituiertes Phenyl, Pyridyl, Thienyl oder Furanyl stehen, wobei als Substituenten in Frage kommen: Alkyl, Alkoxy und Alkylthio mit jeweils 1 oder 2 Kohlenstoffatomen; Halogenalkyl, Halogenalkoxy und Halogenalkylthio mit 1 oder 2

Kohlenstoffatomen und 1 bis 5 Halogenatomen, wobei die Halogenatome gleich oder verschieden sind; Hydroxy; Fluor, Chlor, Brom und Jod; Cyano; Nitro; Dialkylamino mit 1 oder 2 Kohlenstoffatomen je Alkylgruppe; Carboxy; Alkylalkoxy mit 1 oder 2 Kohlenstoffatomen in jedem Alkylteil; Carbonylalkoxy mit 1 oder 2 Kohlenstoffatomen im Alkylteil; Carbonylalkyl mit 1 oder 2 Kohlenstoffatomen im Alkylteil; Formyl; Carbonylphenoxy; Benzoyl; Oxycarbonylalkyl mit 1 oder 2 Kohlenstoffatomen; Benzoyloxy; Carbonylamino, Carbonylaminoalkyl, Carbonylaminodialkyl, Aminocarbonyl, Alkylaminocarbonyl, Aminocarbonylalkyl und Alkylaminocarbonylalkyl mit jeweils 1 oder 2 Kohlenstoffatomen im Alkylteil; Sulfonamido; Sulfonalkyl, Sulfonylalkyl und Sulfonylalkoxy mit jeweils 1 oder 2 Kohlenstoffatomen; jeweils unsubstituiertes oder durch Fluor, Chlor oder Brom substituiertes Phenyl oder Phenoxy,

ausgenommen die Verbindung Carbaminsäure-[1-[[(diphenylmethyl)-amino]-carbonyl]-2-methylpropyl]-1,1-dimethylethylester.

**5.** Verbindungen der Formel (I) gemäß Anspruch 2, in denen

$R^1$ für Methyl, Ethyl, n- und i-Propyl, n-, i-, s- und t-Butyl, Fluormethyl, Fluorethyl, Fluorpropyl, Chlorpropyl, Fluorbutyl, Chlorbutyl, Difluormethyl, Difluorpropyl, Dichlorpropyl, Difluorbutyl, Dichlorbutyl, Trifluormethyl, Trichlormethyl, Trifluorethyl, Trichlorethyl, Trifluorpropyl, Trichlorpropyl, Trifluorbutyl, Trichlorbutyl, Allyl, Butenyl, Propargyl, Butinyl, Fluor- oder Chlor-allyl, -butenyl, -propargyl, -butinyl, Cyclopropyl, Cyclopentyl, Cyclohexyl, Cyclopentenyl oder Cyclohexenyl, für unsubstituiertes oder einfach bis zweifach, gleich oder verschieden substituiertes Phenyl steht, wobei die folgenden Substituenten in Frage kommen: Methyl, Ethyl, n- und i-Propyl, n-, i-, s- und t-Butyl, Methoxy, Ethoxy, i- und n-Propoxy, n-, i-, s- und t-Butoxy, Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Chlor, Brom, Fluor, und

$R^2$ und $R^5$ für Wasserstoff stehen und

$R^3$ und $R^4$ gleich oder verschieden sind und für Wasserstoff, Methyl, Ethyl, n- oder i-Propyl, n-, i-, s- oder t-Butyl, 3-Pentyl, Cyclopropyl, Cyclopentyl oder Cyclohexyl stehen oder

$R^3$ und $R^4$ zusammen mit dem Kohlenstoffatom, an das sie gebunden sind, einen Cyclopropyl-, Cyclopentyl- oder Cyclohexylring bilden und

$R^6$ für Wasserstoff, Methyl, Ethyl, n- und iso-Propyl steht und

$R^7$ und $R^8$ gleich oder verschieden sind und jeweils unabhängig voneinander für jeweils unsubstituiertes oder substituiertes Phenyl, Pyridyl, Furanyl oder Thienyl stehen, wobei als Phenylsubstituenten Methyl, Ethyl, n- und i-Propyl, n-, i-, s- und t-Butyl, Methoxy, Ethoxy, i- und n-Propoxy, n-, i-, s- und t-Butoxy, Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Chlor, Brom, Fluor, Nitro und Cyano und als Heteroarylsubstituenten Chlor, Fluor, Brom und Methyl in Frage kommen,

ausgenommen die Verbindung Carbaminsäure-[1-[[(diphenylmethyl)-amino]-carbonyl]-2-methylpropyl]-1,1-dimethylethylester.

**6.** Verfahren zur Herstellung von Aminosäureamid-Derivate der allgemeinen Formel (I)

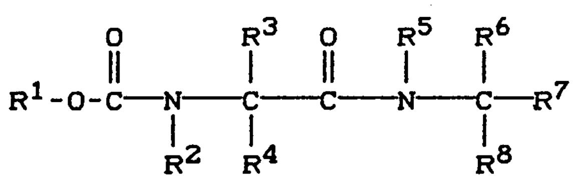

(I),

in welcher

$R^1$, $R^2$, $R^3$, $R^4$, $R^5$, $R^6$, $R^7$ und $R^8$ die in Anspruch

2 angegebene Bedeutung haben, dadurch gekennzeichnet, daß man eine substituierte Aminosäure der Formel (II)

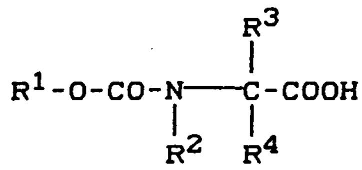

(II),

in welcher

$R^1$, $R^2$, $R^3$ und $R^4$ die oben angegebene Bedeutung haben, bzw. deren carboxy-aktivierte Derivate, gegebenenfalls in Gegenwart eines Katalysators, gegebenenfalls in Gegenwart eines Säurebindemittels und gegebenenfalls in Gegenwart eines Verdünnungsmittels
mit einem Amin der Formel (III)

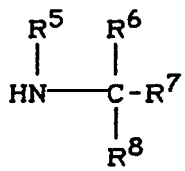

(III)

in welcher
$R^5$, $R^6$, $R^7$ und $R^8$ die oben angegebene Bedeutung haben,
umsetzt.

**7.** Schädlingsbekämpfungsmittel, gekennzeichnet durch einen Gehalt an mindestens einem Aminosäureamid-Derivat der Formel (I) nach den Ansprüchen 1 bis 6.

**8.** Verwendung von Aminosäureamid-Derivaten der Formel (I) nach den Ansprüchen 1 bis 6 zur Bekämpfung von Schädlingen.

**9.** Verfahren zur Bekämpfung von Schädlingen, dadurch gekennzeichnet, daß man Aminosäureamid-Derivate der Formel (I) nach den Ansprüchen 1 bis 6 auf Schädlinge und/oder ihren Lebensraum einwirken läßt.

**10.** Verfahren zur Herstellung von Schädlingsbekämpfungsmitteln, dadurch gekennzeichnet, daß man Aminosäureamid-Derivate der Formel (I) nach den Ansprüchen 1 bis 6 mit Streckmitteln und/oder oberflächenaktiven Mitteln vermischt.

## Claims

**1.** Use of the amino acid amide derivatives of the general formula (I)

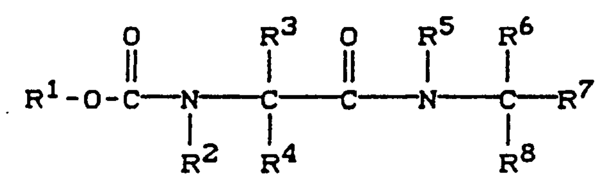

(I).

in which

| | |
|---|---|
| $R^1$ | represents alkyl, alkenyl, alkinyl, halogenoalkyl, halogenoalkenyl, halogenoalkinyl, cycloalkyl, cycloalkenyl, unsubstituted or substituted aryl and unsubstituted or substituted heteroaryl, |
| $R^2$, $R^5$ and $R^6$ | are identical or different and represent hydrogen or alkyl, |
| $R^3$ and $R^4$ | represent hydrogen, cycloalkyl or alkyl, or |
| $R^3$ and $R^4$ | together with the carbon atom to which they are bonded form a cycloalkyl ring |

and

$R^7$ and $R^8$ are identical or different and in each case independently of one another represent phenyl or heteroaryl, each of which is unsubstituted or substituted,

as fungicides.

**2.** Amino acid amide derivatives of the general formula (I)

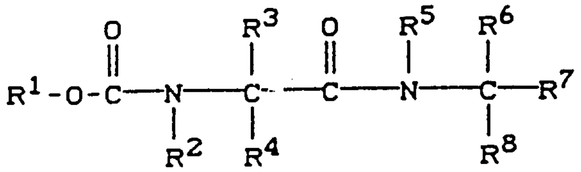

(I),

in which

$R^1$ represents alkyl, alkenyl, alkinyl, halogenoalkyl, halogenoalkenyl, halogenoalkinyl, cycloalkyl, cycloalkenyl, unsubstituted or substituted aryl and unsubstituted or substituted heteroaryl,

$R^2$, $R^5$ and $R^6$ are identical or different and represent hydrogen or alkyl,

$R^3$ and $R^4$ represent hydrogen, cycloalkyl or alkyl, or

$R^3$ and $R^4$ together with the carbon atom to which they are bonded form a cycloalkyl ring and

$R^7$ and $R^8$ are identical or different and in each case independently of one another represent phenyl or heteroaryl, each of which is unsubstituted or substituted, with the exception of the compound [1-[[(diphenylmethyl)-amino]carbonyl]-2-methylpropyl]-1,1-dimethylethyl carbamate.

**3.** Compounds of the formula (I) according to Claim 2, in which

$R^1$ represents straight-chain or branched alkyl having 1 to 6 carbon atoms, straight-chain or branched alkenyl or alkinyl having 2 to 6 carbon atoms, straight-chain or branched halogenoalkyl having 1 to 6 carbon atoms and 1 to 9 identical or different halogen atoms, straight-chain or branched halogenoalkenyl or halogenoalkinyl having 2 to 6 carbon atoms and 1 to 9 identical or different halogen atoms, or substituted or unsubstituted cycloalkyl or cycloalkenyl having 3 to 7 carbon atoms, or represents phenyl or pyridyl, each of which is unsubstituted or substituted, suitable substituents in the pyridyl or phenyl moiety in each case being: alkyl, alkoxy and alkylthio, each of which has 1 to 4 carbon atoms; halogenoalkyl, halogenoalkoxy and halogenoalkylthio, each of which has 1 to 4 carbon atoms and 1 to 9 halogen atoms, the halogen atoms being identical or different; halogen; alkylalkoxy having 1 to 4 carbon atoms in each alkyl moiety; carbonylalkoxy having 1 to 4 carbon atoms in the alkyl moiety;

$R^2$ and $R^5$ represent hydrogen or methyl

$R^3$ and $R^4$ are identical or different and represent hydrogen, unsubstituted or substituted cycloalkyl having 3 to 7 carbon atoms, or straight chain or branched alkyl having 1 to 6 carbon atoms or

$R^3$ and $R^4$ together with the carbon atom to which they are bonded form a cycloalkyl ring having 3 to 7 carbon atoms,

$R^6$ represents hydrogen or branched or unbranched alkyl having 1 to 6 carbon atoms and

$R^7$ and $R^8$ are identical or different and in each case represent unsubstituted or substituted phenyl, pyridyl, furanyl or thienyl, suitable substituents in each case being:
alkyl, alkoxy and alkylthio, each of which has 1 to 4 carbon atoms; halogenoalkyl, halogenoalkoxy and halogenoalkylthio, each of which has 1 to 4 carbon atoms and 1 to 9 halogen atoms, the halogen atoms being identical or different; hydroxyl; halogen; cyano; nitro; dialkylamino having 1 to 4 carbon atoms per alkyl group; carboxyl; alkylalkoxy having 1 to 4 carbon atoms in each alkyl moiety; carbonylalkoxy having 1 to 4 carbon atoms in the alkyl moiety; carbonylalkyl having 1 to 4 carbon atoms in the alkyl moiety; formyl; carbonylaryloxy having 6 to 10 carbon atoms in the aryl moiety;

carbonylaryl having 6 to 10 carbon atoms in the aryl moiety; oxycarbonylalkyl having 1 to 4 carbon atoms in the alkyl moiety; oxycarbonylaryl having 6 to 10 carbon atoms in the aryl moiety; carbonylamino, carbonylaminoalkyl, carbonylaminodialkyl, aminocarbonyl, alkylaminocarbonyl, aminocarbonylalkyl and alkylaminocarbonylalkyl, each of which has 1 to 4 carbon atoms in the alkyl moiety; sulphonamido; sulphonylalkyl and sulphonylalkoxy, each of which has 1 to 4 carbon atoms; phenyl or phenoxy, each of which is unsubstituted or substituted by halogen,

with the exception of the compound [1-[[(diphenylmethyl)-amino]carbonyl]-2-methylpropyl]-1,1-dimethylethyl carbamate.

**4.** Compounds of the formula (I) according to Claim 2, in which

$R^1$ represents straight-chain or branched alkyl having 1 to 4 carbon atoms, straight-chain or branched alkenyl or alkinyl having 2 to 4 carbon atoms, straight-chain or branched halogenoalkyl having 1 to 4 carbon atoms and 1 to 5 identical or different halogen atoms, straight-chain or branched halogenoalkenyl or halogenoalkinyl having 2 to 4 carbon atoms and 1 to 5 identical or different halogen atoms, or unsubstituted or substituted cycloalkyl or cycloalkenyl having 3 to 6 carbon atoms, and furthermore represents phenyl which is unsubstituted or monosubstituted to trisubstituted by identical or different substituents, suitable substituents being the following: alkyl, alkoxy and alkylthio, each of which has 1 to 4 carbon atoms; halogenoalkyl, halogenoalkoxy and halogenoalkylthio, each of which has 1 or 2 carbon atoms and 1 to 5 halogen atoms, the halogen atoms being identical or different; fluorine, chlorine, bromine and iodine; alkylalkoxy having 1 to 2 carbon atoms in each alkyl moiety; carbonylalkoxy having 1 or 2 carbon atoms in the alkyl moiety;

$R^2$ and $R^5$ represent hydrogen,

$R^3$ and $R^4$ are identical or different and represent hydrogen unsubstituted or substituted cycloalkyl having 3 to 6 carbon atoms or straight-chain or branched alkyl having 1 to 5 carbon atoms or

$R^3$ and $R^4$ together with the carbon atom to which they are bonded form a cycloalkyl ring having 3 to 6 carbon atoms

$R^6$ represents hydrogen or branched or unbranched alkyl having 1 to 4 carbon atoms and

$R^7$ and $R^8$ are identical or different and in each case independently of one another represent phenyl, pyridyl, thienyl or furanyl, each of which is unsubstituted or substituted, suitable substituents being: alkyl, alkoxy and alkylthio, each of which has 1 or 2 carbon atoms; halogenoalkyl, halogenoalkoxy and halogenoalkylthio having 1 or 2 carbon atoms and 1 to 5 halogen atoms, the halogen atoms being identical or different; hydroxyl; fluorine, chlorine, bromine and iodine; cyano; nitro; dialkylamino having 1 or 2 carbon atoms per alkyl group; carboxyl; alkylalkoxy having 1 or 2 carbon atoms in each alkyl moiety; carbonylalkoxy having 1 or 2 carbon atoms in the alkyl moiety; carbonylalkyl having 1 or 2 carbon atoms in the alkyl moiety; formyl; carbonylphenoxy; benzoyl; oxycarbonylalkyl having 1 or 2 carbon atoms; benzoyloxy; carbonylamino, carbonylaminoalkyl, carbonylaminodialkyl, aminocarbonyl, alkylaminocarbonyl, aminocarbonylalkyl and alkylaminocarbonylalkyl, each of which has 1 or 2 carbon atoms in the alkyl moiety; sulphonamido; sulphonalkyl, sulphonylalkyl and sulphonylalkoxy, each of which has 1 or 2 carbon atoms; phenyl or phenoxy, each of which is unsubstituted or substituted by fluorine, chlorine or bromine,

with the exception of the compound [1-[[(diphenylmethyl)-amino]-carbonyl]-2-methylpropyl]-1,1-dimethylethyl carbamate.

**5.** Compounds of the formula (I) according to Claim 2, in which

$R^1$ represents methyl, ethyl, n- and i-propyl, n-, i-, s- and t-butyl, fluoromethyl, fluoroethyl, fluoropropyl, chloropropyl, fluorobutyl, chlorobutyl, difluoromethyl, difluoropropyl, dichloropropyl, difluorobutyl, dichlorobutyl, trifluoromethyl, trichloromethyl, trifluoroethyl, trichloroethyl, trifluoropropyl, trichloropropyl, trifluorobutyl, trichlorobutyl, allyl, butenyl, propargyl, butinyl, fluoro- or chloroallyl, -butenyl, -propargyl, -butinyl, cyclopropyl, cyclopentyl, cyclohexyl, cyclopentenyl or cyclohexenyl, or represents phenyl which is unsubstituted or monosubstituted to disubstituted by

identical or different substituents, suitable substituents being the following: methyl, ethyl, n- and i-propyl, n-, i-, s- and t-butyl, methoxy, ethoxy, i- and n-propoxy, n-, i-, s- and t-butoxy, trifluoromethyl, difluoromethyl, pentafluoroethyl, tetrafluoroethyl, trifluorochloroethyl, trifluoroethyl, trifluoroethoxy, difluoromethoxy, pentafluoroethoxy, tetrafluoroethoxy, trifluorochloroethoxy, trifluoromethoxy and trifluoromethylthio; chlorine, bromine, fluorine, and

$R^2$ and $R^5$ represent hydrogen and

$R^3$ and $R^4$ are identical or different and represent hydrogen, methyl, ethyl, n- or i-propyl, n-, i-, s- or t-butyl, 3-pentyl, cyclopropyl, cyclopentyl or cyclohexyl or

$R^3$ and $R^4$ together with the carbon atom to which they are bonded form a cyclopropyl, cyclopentyl or cyclohexyl ring and

$R^6$ represents hydrogen, methyl, ethyl, n- and isopropyl and

$R^7$ and $R^8$ are identical or different and in each case independently of one another represent phenyl, pyridyl, furanyl or thienyl, each of which is unsubstituted or substituted, suitable phenyl substitutents being methyl, ethyl, n- and i-propyl, n-, i-, s- and t-butyl, methoxy, ethoxy, i- and n-propoxy, n-, i-, s- and t-butoxy, trifluoromethyl, difluoromethyl, pentafluoroethyl, tetrafluoroethyl, trifluorochloroethyl, trifluoroethyl, trifluoroethoxy, difluoromethoxy, pentafluoroethoxy, tetrafluoroethoxy, trifluorochloroethoxy, trifluoromethoxy and trifluoromethylthio; chlorine, bromine, fluorine, nitro and cyano, and suitable heteroaryl substituents being chlorine, fluorine, bromine and methyl,

with the exception of the compound [1-[[(diphenylmethyl)-amino]-carbonyl]-2-methylpropyl]-1,1-dimethylethyl carbamate.

**6.** Process for the preparation of amino acid amide derivatives of the general formula (I)

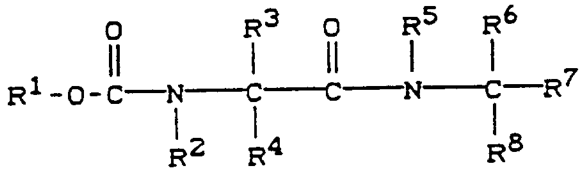

(I),

in which
$R^1$, $R^2$, $R^3$, $R^4$, $R^5$, $R^6$, $R^7$ and $R^8$ have the meaning given in Claim 2,
characterised in that a substituted amino acid of the formula (II)

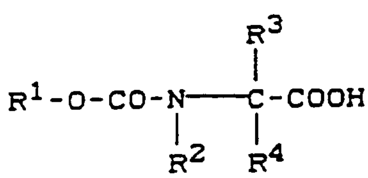

(II),

in which
$R^1$, $R^2$, $R^3$ and $R^4$ have the abovementioned meaning,
or their carboxyl-activated derivatives,
are reacted with an amine of the formula (III)

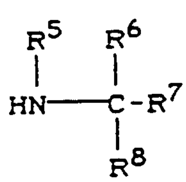

(III)

in which
$R^5$, $R^6$, $R^7$ and $R^8$ have the abovementioned meaning,
if appropriate in the presence of a catalyst, if appropriate in the presence of an acid-binding agent and if appropriate in the presence of a diluent.

7. Pesticides, characterised in that they contain at least one amino acid amide derivative of the formula (I) according to Claims 1 to 6.

8. Use of amino acid amide derivatives of the formula (I) according to Claims 1 to 6 for combating pests.

9. Method of combating pests, characterised in that amino acid amide derivatives of the formula (I) according to Claims 1 to 6 are allowed to act on pests and/or their environment.

10. Process for the preparation of pesticides, characterised in that amino acid amide derivatives of the formula (I) according to Claims 1 to 6 are mixed with extenders and/or surface-active agents.

**Revendications**

1. Utilisation des dérivés d'amides d'aminoacides répondant à la formule générale (I)

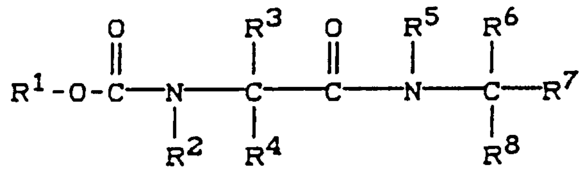

(I).

dans laquelle

| | |
|---|---|
| $R^1$ | représente un groupe alkyle, un groupe alcényle, un groupe alcynyle, un groupe halogénalkyle, un groupe halogénalcényle, un groupe halogénalcynyle, un groupe cycloalkyle, un groupe cycloalcényle, un groupe aryle non substitué ou substitué et un groupe hétéroaryle non substitué ou substitué, |
| $R^2$, $R^5$ et $R^6$ | sont identiques ou différents et représentent un atome d'hydrogène ou un groupe alkyle, |
| $R^3$ et $R^4$ | représentent un atome d'hydrogène, un groupe cycloalkyle ou un groupe alkyle, ou |
| $R^3$ et $R^4$, | ensemble avec l'atome de carbone auquel ils sont liés, forment un noyau cycloalkyle, et |
| $R^7$ et $R^8$ | sont identiques ou différents et représentent, chacun indépendamment l'un de l'autre, un groupe phényle ou un groupe hétéroaryle, respectivement non substitué ou substitué, |

comme fongicides.

2. Dérivés d'amides d'aminoacides répondant à la formule générale (I)

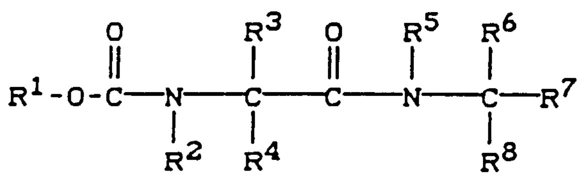

(I),

dans laquelle

| | |
|---|---|
| $R^1$ | représente un groupe alkyle, un groupe alcényle, un groupe alcynyle, un groupe halogénalkyle, un groupe halogénalcényle, un groupe halogénalcynyle, un groupe cycloalkyle, un groupe cycloalcényle, un groupe aryle non substitué ou substitué et |

un groupe hétéroaryle non substitué ou substitué,

$R^2$, $R^5$ et $R^6$ sont identiques ou différents et représentent un atome d'hydrogène ou un groupe alkyle,

$R^3$ et $R^4$ représentent un atome d'hydrogène, un groupe cycloalkyle ou un groupe alkyle, ou

$R^3$ et $R^4$, ensemble avec l'atome de carbone auquel ils sont liés, forment un noyau cycloalkyle, et

$R^7$ et $R^8$ sont identiques ou différents et représentent, chacun indépendamment l'un de l'autre, un groupe phényle ou un groupe hétéroaryle, respectivement non substitué ou substitué,

à l'exception du composé ester [1-[[(diphénylméthyl)-amino]carbonyl]-2-méthylpropyl]-1,1-diméthyléthylique de l'acide carbamique.

**3.** Composés de formule (I) selon la revendication 2, dans laquelle

$R^1$ représente un groupe alkyle à chaîne droite ou ramifiée contenant de 1 à 6 atomes de carbone, un groupe alcényle ou un groupe alcynyle à chaîne droite ou ramifiée contenant de 2 à 6 atomes de carbone, un groupe halogénalkyle à chaîne droite ou ramifiée contenant de 1 à 6 atomes de carbone et de 1 à 9 atomes d'halogène identiques ou différents, un groupe halogénalcényle ou un groupe halogénalcynyle à chaîne droite ou ramifiée contenant de 2 à 6 atomes de carbone et de 1 à 9 atomes d'halogène identiques ou différents, ou encore un groupe cycloalkyle ou un groupe cycloalcényle substitué ou non substitué, contenant de 3 à 7 atomes de carbone, un groupe phényle et un groupe pyridyle, respectivement non substitué ou substitué, dans lesquels, comme substituants dans la fraction pyridyle, respectivement phényle, on envisage respectivement : un groupe alkyle, un groupe alcoxy et un groupe alkylthio contenant respectivement de 1 à 4 atomes de carbone; un groupe halogénalkyle, un groupe halogénalcoxy et un groupe halogénalkylthio contenant respectivement de 1 à 4 atomes de carbone et de 1 à 9 atomes d'halogène, les atomes d'halogène étant identiques ou différents; un atome d'halogène; un groupe alkylalcoxy contenant de 1 à 4 atomes de carbone dans chaque fraction alkyle; un groupe carbonylalcoxy contenant de 1 à 4 atomes de carbone dans la fraction alkyle;

$R^2$ et $R^5$ représentent un atome d'hydrogène ou un groupe méthyle,

$R^3$ et $R^4$ sont identiques ou différents et représentent un atome d'hydrogène, un groupe cycloalkyle non substitué ou substitué contenant de 3 à 7 atomes de carbone, ou encore un groupe alkyle à chaîne droite ou ramifiée contenant de 1 à 6 atomes de carbone, ou

$R^3$ et $R^4$, conjointement avec l'atome de carbone auquel ils sont liés, forment un noyau cycloalkyle contenant de 3 à 7 atomes de carbone,

$R^6$ représente un atome d'hydrogène ou un groupe alkyle à chaîne droite ou ramifiée contenant de 1 à 6 atomes de carbone, et

$R^7$ et $R^8$ sont identiques ou différents et représentent un groupe phényle, un groupe pyridyle, un groupe furanyle ou un groupe thiényle, respectivement non substitué ou substitué, dans lesquels, comme substituants, on envisage respectivement :

un groupe alkyle, un groupe alcoxy et un groupe alkylthio contenant respectivement de 1 à 4 atomes de carbone; un groupe halogénalkyle, un groupe halogénalcoxy et un groupe halogénalkylthio contenant respectivement de 1 à 4 atomes de carbone et de 1 à 9 atomes d'halogène, les atomes d'halogène étant identiques ou différents; un groupe hydroxyle; un atome d'halogène; un groupe cyano; un groupe nitro; un groupe dialkylamino contenant de 1 à 4 atomes de carbone par groupe alkyle; un groupe carboxyle; un groupe alkylalcoxy contenant de 1 à 4 atomes de carbone dans chaque fraction alkyle; un groupe carbonylalcoxy contenant de 1 à 4 atomes de carbone dans la fraction alkyle; un groupe carbonylalkyle contenant de 1 à 4 atomes de carbone dans la fraction alkyle; un groupe formyle; un groupe carbonylaryloxy contenant de 6 à 10 atomes de carbone dans la fraction aryle; un groupe carbonylaryle contenant de 6 à 10 atomes de carbone dans la fraction aryle; un groupe oxycarbonylalkyle contenant de 1 à 4 atomes de carbone dans la fraction alkyle; un groupe oxycarbonylaryle contenant de 6 à 10 atomes de carbone dans la fraction aryle; un groupe carbonylamino, un groupe carbonylaminoalkyle, un groupe carbonylaminodialkyle, un groupe aminocarbonyle, un groupe alkylaminocarbonyle, un groupe aminocarbonylalkyle et un groupe alkylaminocarbonylalkyle contenant respectivement de 1 à 4 atomes de carbone dans la fraction alkyle; un groupe sulfonamido; un groupe sulfonylalkyle et un groupe sulfonylalcoxy contenant respectivement de 1 à 4 atomes de carbone; un groupe phényle ou un groupe phénoxy, respectivement non substitué ou substitué par un atome d'halogène,

à l'exception du composé ester [1-[[(diphénylméthyl)-amino]carbonyl]-2-méthylpropyl]-1,1-diméthyléthylique de l'acide carbamique.

**4.** Composés de formule (I) selon la revendication 2, dans laquelle

$R^1$ représente un groupe alkyle à chaîne droite ou ramifiée contenant de 1 à 4 atomes de carbone, un groupe alcényle ou un groupe alcynyle à chaîne droite ou ramifiée contenant de 2 à 4 atomes de carbone, un groupe halogénalkyle à chaîne droite ou ramifiée contenant de 1 à 4 atomes de carbone et de 1 à 5 atomes d'halogène identiques ou différents, un groupe halogénalcényle ou un groupe halogénalcynyle à chaîne droite ou ramifiée contenant de 2 à 4 atomes de carbone et de 1 à 5 atomes d'halogène identiques ou différents, ou encore un groupe cycloalkyle ou un groupe cycloalcényle substitué ou non substitué, contenant de 3 à 6 atomes de carbone, et en outre, un groupe phényle non substitué ou substitué de 1 à 3 fois de manière identique ou différente, dans lequel on envisage les substituants ci-après : un groupe alkyle, un groupe alcoxy et un groupe alkylthio contenant respectivement de 1 à 4 atomes de carbone; un groupe halogénalkyle, un groupe halogénalcoxy et un groupe halogénalkylthio contenant respectivement 1 ou 2 atomes de carbone et de 1 à 5 atomes d'halogène, les atomes d'halogène étant identiques ou différents; un atome de fluor, un atome de chlore, un atome de brome et un atome d'iode; un groupe alkylalcoxy contenant de 1 à 2 atomes de carbone dans chaque fraction alkyle; un groupe carbonylalcoxy contenant 1 ou 2 atomes de carbone dans la fraction alkyle;

$R^2$ et $R^5$ représentent un atome d'hydrogène,

$R^3$ et $R^4$ sont identiques ou différents et représentent un atome d'hydrogène, un groupe cycloalkyle non substitué ou substitué contenant de 3 à 6 atomes de carbone, ou encore un groupe alkyle à chaîne droite ou ramifiée contenant de 1 à 5 atomes de carbone, ou

$R^3$ et $R^4$, conjointement avec l'atome de carbone auquel ils sont liés, forment un noyau cycloalkyle contenant de 3 à 6 atomes de carbone,

$R^6$ représente un atome d'hydrogène ou un groupe alkyle à chaîne droite ou ramifiée contenant de 1 à 4 atomes de carbone, et

$R^7$ et $R^8$ sont identiques ou différents et représentent, chacun indépendamment l'un de l'autre, un groupe phényle, un groupe pyridyle, un groupe thiényle ou un groupe furanyle, respectivement non substitué ou substitué, dans lesquels, comme substituants, on envisage :

un groupe alkyle, un groupe alcoxy et un groupe alkylthio contenant respectivement 1 ou 2 atomes de carbone; un groupe halogénalkyle, un groupe halogénalcoxy et un groupe halogénalkylthio contenant 1 ou 2 atomes de carbone et de 1 à 5 atomes d'halogène, les atomes d'halogène étant identiques ou différents; un groupe hydroxyle; un atome de fluor, un atome de chlore, un atome de brome et un atome d'iode; un groupe cyano; un groupe nitro; un groupe dialkylamino contenant 1 ou 2 atomes de carbone par groupe alkyle; un groupe carboxyle; un groupe alkylalcoxy contenant 1 ou 2 atomes de carbone dans chaque fraction alkyle; un groupe carbonylalcoxy contenant 1 ou 2 atomes de carbone dans la fraction alkyle; un groupe carbonylalkyle contenant 1 ou 2 atomes de carbone dans la fraction alkyle; un groupe formyle; un groupe carbonylphénoxy; un groupe benzoyle; un groupe oxycarbonylalkyle contenant 1 ou 2 atomes de carbone dans la fraction alkyle; un groupe benzoyloxy; un groupe carbonylamino, un groupe carbonylaminoalkyle, un groupe carbonylaminodialkyle, un groupe aminocarbonyle, un groupe alkylaminocarbonyle, un groupe aminocarbonylalkyle et un groupe alkylaminocarbonylalkyle contenant respectivement 1 ou 2 atomes de carbone dans la fraction alkyle; un groupe sulfonamido; un groupe sulfonalkyle, un groupe sulfonylalkyle et un groupe sulfonylalcoxy contenant respectivement 1 ou 2 atomes de carbone; un groupe phényle ou un groupe phénoxy, respectivement non substitué ou substitué par un atome de fluor, par un atome de chlore ou par un atome de brome,

à l'exception du composé ester [1-[[(diphénylméthyl)-amino]carbonyl]-2-méthylpropyl]-1,1-diméthyléthylique de l'acide carbamique.

5. Composés de formule (I) selon la revendication 2, dans lesquels

$R^1$ représente un groupe méthyle, un groupe éthyle, un groupe n- et i-propyle, un groupe n-, i-, s- et t-butyle, un groupe fluorométhyle, un groupe fluoréthyle, un groupe fluoropropyle, un groupe chloropropyle, un groupe fluorobutyle, un groupe chlorobutyle, un groupe difluorométhyle, un groupe difluoropropyle, un groupe dichloropropyle, un groupe difluorobutyle, un groupe dichlorobutyle, un groupe trifluorométhyle, un groupe trichlorométhyle, un groupe trifluoréthyle, un groupe trichloréthyle, un groupe trifluoropropyle, un groupe trichloropropyle, un groupe trifluorobutyle, un groupe trichlorobutyle, un groupe allyle, un groupe butényle, un groupe propargyle, un groupe butynyle, un groupe fluoro- ou chloro-allyle, -butényle, -propargyle, -butynyle, un groupe cyclopropyle, un groupe cyclopentyle, un groupe cyclohexyle, un groupe cyclopentényle ou un groupe cyclohexényle; un groupe phényle non substitué ou substitué de 1 à 2 fois de manière identique ou différente, dans lequel on envisage les substituants ci-après : un groupe méthyle, un groupe éthyle, un groupe n- et i-propyle, un groupe n-, i-, s- et t-butyle, un groupe méthoxy, un groupe éthoxy, un groupe i- et n-propoxy, un groupe n-, i-, s-et t-butoxy, un groupe trifluorométhyle, un groupe difluorométhyle, un groupe pentafluoréthyle, un groupe tétrafluoréthyle, un groupe trifluorochloréthyle, un groupe trifluoréthyle, un groupe trifluoréthoxy, un groupe difluorométhoxy, un groupe pentafluoréthoxy, un groupe tétrafluoréthoxy, un groupe trifluorochloréthoxy, un groupe trifluorométhoxy et un groupe trifluorométhylthio; un atome de chlore, un atome de brome, un atome de fluor, et

$R^2$ et $R^5$ représentent un atome d'hydrogène et

$R^3$ et $R^4$ sont identiques ou différents et représentent un atome d'hydrogène, un groupe méthyle, un groupe éthyle, un groupe n- ou i-propyle, un groupe n-, i-, s- ou t-butyle, un groupe 3-pentyle, un groupe cyclopropyle, un groupe cyclopentyle ou un groupe cyclohexyle, ou

$R^3$ et $R^4$, ensemble avec l'atome de carbone auquel ils sont liés, forment un noyau cyclopropyle, cyclopentyle ou cyclohexyle, et

$R^6$ représente un atome d'hydrogène, un groupe méthyle, un groupe éthyle, un groupe n- et isopropyle, et

$R^7$ et $R^8$ sont identiques ou différents et représentent, chacun indépendamment l'un de l'autre, un groupe phényle, un groupe pyridyle, un groupe furanyle ou un groupe thiényle, respectivement non substitué ou substitué, dans lesquels, comme substituants du groupe phényle, on envisage : un groupe méthyle, un groupe éthyle, un groupe n- et i-propyle, un groupe n-, i-, s-et t-butyle, un groupe méthoxy, un groupe éthoxy, un groupe i- et n-propoxy, un groupe n-, i-, s- et t-butoxy, un groupe trifluorométhyle, un groupe difluorométhyle, un groupe pentafluoréthyle, un groupe tétrafluoréthyle, un groupe trifluorochloréthyle, un groupe trifluoréthyle, un groupe trifluoréthoxy, un groupe difluorométhoxy, un groupe pentafluoréthoxy, un groupe tétrafluoréthoxy, un groupe trifluorochloréthoxy, un groupe trifluorométhoxy et un groupe trifluorométhylthio; un atome de chlore, un atome de brome, un atome de fluor, un groupe nitro et un groupe cyano, et comme substituants du groupe hétéroaryle, un atome de chlore, un atome de fluor, un atome de brome et un groupe méthyle,

à l'exception du composé ester [1-[[(diphénylméthyl)-amino]carbonyl]-2-méthylpropyl]-1,1-diméthyléthylique de l'acide carbamique.

6. Procédé pour la préparation de dérivés d'amides d'aminoacides répondant à la formule générale (I)

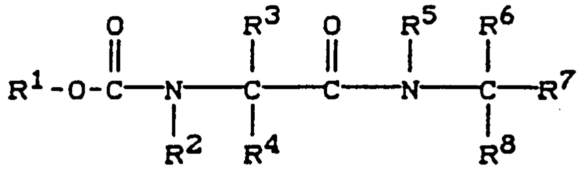

(I),

dans laquelle

$R^1$, $R^2$, $R^3$, $R^4$, $R^5$, $R^6$, $R^7$ et $R^8$ ont la signification indiquée à la revendication 2,

caractérisé en ce qu'on fait réagir un aminoacide substitué de formule (II)

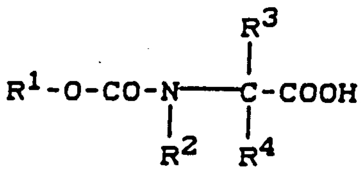

(II),

dans laquelle
$R^1$, $R^2$, $R^3$ et $R^4$ ont la signification indiquée ci-dessus,
respectivement ses dérivés activés par un groupe carboxyle,
éventuellement en présence d'un catalyseur, éventuellement en présence d'un agent neutralisateur d'acides et éventuellement en présence d'un diluant,
avec une amine de formule (III)

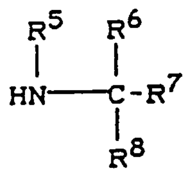

(III)

dans laquelle
$R^5$, $R^6$, $R^7$ et $R^8$ ont la signification indiquée ci-dessus.

7. Agent de lutte contre les parasites, caractérisé par une teneur en au moins un dérivé d'amide d'aminoacide de formule (I) selon les revendications 1 à 6.

8. Utilisation de dérivés d'amides d'aminoacides de formule (I) selon les revendications 1 à 6, pour lutter contre les parasites.

9. Procédé pour lutter contre les parasites, caractérisé en ce qu'on laisse agir des dérivés d'amides d'aminoacides de formule (I) selon les revendications 1 à 6, sur des parasites et/ou sur leur biotope.

10. Procédé pour la préparation d'agents de lutte contre les parasites, caractérisé en ce qu'on mélange des dérivés d'amides d'aminoacides de formule (I) selon les revendications 1 à 6, avec des diluants et/ou des agents tensioactifs.